# EUROPEAN PATENT SPECIFICATION

(11) **EP 1 491 890 B1**
(45) Date of publication and mention of the grant of the patent: **29.07.2009**
(21) Application number: 04700512.9
(22) Date of filing: 07.01.2004
(51) Int. Cl.: C07K 16/18, G01N 33/53, G01N 33/68

(54) **METHOD OF EVALUATING HAIR GROWTH-PROMOTING ACTIVITY**
VERFAHREN ZUR BEURTEILUNG EINER HAARWACHSTUMSFÖRDERNDEN AKTIVITÄT
PROCEDE D'EVALUATION D'UNE ACTIVITE FAVORISANT LA POUSSE DES CHEVEUX

(30) Priority: 08.01.2003 JP 2003001891
(43) Date of publication of application: 29.12.2004
(73) Proprietor: Sumitomo Electric Industries, Ltd., Osaka-shi, Osaka 541-0041 (JP)
(72) Inventor: TAKEBE, Kyoko, c/o Sumitomo Electric Ind.,Ltd., Kanagawa 244-8588 (JP); HIRAI, Yohei, c/o Sumitomo Electric Ind.,Ltd., Yokohama-shi, Kanagawa 244-8588 (JP)
(74) Representative: Polypatent
(86) International application number: PCT/JP2004/000030
(87) International publication number: WO 2004/063750

(56) References cited:
- EP-A- 1 156 334
- EP-A- 1 310 511
- JP-A- 2001 286 299
- JP-A- 2001 318 096
- JP-A- 2003 210 169
- TAKEBE, K., ET AL: "Epimorphin acts to induce hair follicle anagen in C57BL/6 mice" THE FASEB JOURNAL, vol. 17, November 2003 (2003-11), pages 2037-2047, XP002362090

## Description

### Technical Field

This invention relates to a method for the evaluation of hair growth promoting activity using an antibody which is specific for epithelial new follicle.

### Background Art

The normal morphogenesis of epithelial tissue has been suggested to be controlled by factors derived from mesenchymal cells present around the epithelial tissue. Diseases resulting from the abnormal morphogenesis of epithelial tissue are largely caused by abnormalities of mesenchymal cells. Therefore, an interest has arisen in the clarification of the mechanism in which mesenchymal cells controls the morphogenesis of epithelial tissue. However, substances involved in the control of morphogenesis of epithelial tissue are expressed under time and spatial control in a complicated system, and accordingly, it is extremely difficult to isolate these substances and analyze their functions. It is also difficult to construct a model experimental system simplifying the morphogenesis of epithelial tissue. For these reasons, no significant progress has been made to date in researches in this field. Thus, analysis of the controlling mechanism for the morphogenesis of epithelial tissue has been highly desired in order to elucidate the mechanism of occurrence of diseases associated with the morphogenesis of epithelial tissue and establish methods for treating these diseases.

Under the circumstances, epimorphin involved in the control of the morphogenesis of epithelial tissue was isolated (EP0562123). This substance, being a physiologically active substance containing a protein consisting of 277 to 289 amino acids as a core protein, was revealed to be biosynthesized mainly by mesenchymal cells. It was also found that epimorphin had the action of promoting the morphogenesis of epithelial tissue through its action on epithelial cells, and that normal tissue formation was not progressed when epimorphin failed to function.

As to the structure of epimorphin, it has been found that epimorphin molecule can be roughly divided into four fragments from a structural viewpoint (European Patent Publication No. 0698666). That is, the polypeptide consisting of full length of epimorphin can be divided into the following four fragments, from its N-terminal, a coiled coil domain (1), a functional domain (2), a coiled coil domain (3), and hydrophobic domain at the C-terminal. In these fragments, it is suggested that the functional domain (the domain specified by 104th to 187th amino acids in human epimorphin) participates in cell adhesion and is closely related with an expression of physiological activity of epimorphin (the above-mentioned EP 0698666).

Since epimorphin has an action for promoting normal morphogenesis, this substance is expected to be useful as an active ingredient of medicaments for preventive or therapeutic treatment of diseases caused by abnormal morphogenesis, or medicaments such as a hair growth promoting agent.

However, a native epimorphin obtained from a mammal is almost insoluble in an aqueous media such as saline, which causes difficulty in practically using the substance as medicaments. Some attempts were made to produce epimorphin derivatives having good solubility while substantially keeping the promoting activity on morphogenesis of the native epimorphin. For example, International Publication WO01/94382 discloses pep7 as an example of such a peptide having a hair growth promoting activity. In such attempts to produce new epimorphin derivatives, it is essential to evaluate a hair growth promoting activity.

A specific method for evaluating a hair growth promoting activity is mentioned below. C3H and C57BL/6 mice are known to have sustained telogen for about 50 days from the 45th day after the birth to around the 95th day. Their hair cycle is easily judged based on the skin color changes, i.e., from pink in telogen to gray or black in anagen. A hair growth promoting activity can be evaluated by using this mice and evaluating whether or not the administration the test substance promotes the transition from telogen to anagen. However, it has been desired to develop a method for more simply and rapidly evaluating a hair growth promoting activity in vitro.

The post-published document EP 1 310 511 A1 of the same applicant describes a monoclonal antibody which specifically recognizes an antigen of about 220 kDa present in epithelial new follicles and to a method for the evaluation of hair growth promoting activity using this monoclonal antibody.

### Disclosure of the Invention

An object of the present invention is to provide a method for the evaluation of hair growth promoting activity using an antibody which is useful for the evaluation of hair growth promoting activity and specifically recognizes a protein present in epithelial new follicle, and a kit for the evaluation of hair growth promoting activity comprising said antibody.

In order to achieve the above-mentioned object, the present inventors have carried out an intensive study, and have succeeded in identifying the amino acid sequence of a protein which is highly expressed at anagen stage of hair cycle and is useful for the judgment of hair growth inducing activity, and simply and rapidly evaluating the hair growth promoting activity by using an antibody against said protein, thereby completing the present invention.

The present invention provides a method for the evaluation of hair growth promoting activity which comprises the steps of:
incubating skin tissue pieces derived from living organism in the presence
of a substance to be tested;
recovering said skin tissue pieces, and reacting them with a polyclonal
antibody which recognizes a protein of amino acid sequence SEQ ID NO: 1 of the sequence listing; and
detecting or measuring said antibody which has reacted with the skin tissue
pieces.

Thus, in accordance with the present invention, there is provided a method for the evaluation of hair growth promoting activity wherein a polyclonal antibody which recognizes a protein of amino acid sequence of SEQ ID NO.1 of the sequence listing is used. The aforementioned polyclonal antibody includes an antibody which is obtained by immunizing an animal other than human with a protein of amino acid sequence of SEQ ID NO.1 of the sequence listing.

There is provided a method for the evaluation of hair growth promoting activity which comprises steps of incubating skin tissue pieces derived from living organism in the presence of a substance to be tested; recovering said skin tissue pieces, and reacting them with a polyclonal antibody which recognizes a protein of amino acid sequence of SEQ ID NO.1 of the sequence listing ; and detecting or measuring said polyclonal antibody which was reacted with the skin tissue pieces. The aforementioned antibody includes a polyclonal antibody which is obtained by immunizing an animal other than human with a protein of amino acid sequence of SEQ ID NO.1 of the sequence listing.

### Brief Description of the Drawings

Fig.1 shows the partial amino acid sequence of the antigen which is recognized by the antibody used in the present invention.
Fig. 2 shows the result of the evaluation of a hair growth promoting activity according to the method of the present invention.

### Best Mode for Carrying out the Invention

The embodiments of the practice of the present invention are mentioned below in detail.

### (1) A method for the evaluation of hair growth promoting activity wherein a polyclonal antibody which recognizes a protein of amino acid sequence of SEQ ID NO.1 of the sequence listing is used

The method for the evaluation of hair growth promoting activity according to the present invention is a method wherein a polyclonal antibody which recognizes a protein of amino acid sequence of SEQ ID NO.1 of the sequence listing is used, and, the hair growth promoting activity is evaluated by incubating skin tissue derived from living organism in the presence of a substance to be tested, recovering said skin tissue pieces, and reacting them with a polyclonal antibody which recognizes a protein of amino acid sequence of SEQ ID NO.1 of the sequence listing ; and detecting or measuring said antibody which was reacted with the skin tissue pieces.

A protein of the amino acid sequence shown in SEQ ID NO: 1 of the sequence listing is registered in the database (NCIB (GeneBank)) under registration No. XM_177952 (*Mus musculus*, similar to trichohyalin). It has a small degree of homology with an inner root sheath protein "trichohyalin" of human hair follicles. The functions of this protein and the expression thereof have been totally unknown until now. It is, however, clarified by the present invention that the expression of this protein is highly specific for hair follicles in anagen. As other proteins highly expressed in hair follicles in anagen, hair keratin and Hacl-1 have been known. A substance having hair growth inducing activity was added to a culture system of skin tissues, and an increase in the expression of these proteins was examined using an antibody. As a result, it was found that the expression of the protein of the present invention was most sensitive (increase in expression) to the addition of the hair growth-inducing substance in the above culture system.

The polyclonal antibody used in the present invention is **characterized in that** it recognizes the protein of the amino acid sequence shown in SEQ ID NO: 1 of the sequence listing. The aforementioned polyclonal antibody includes an antibody which is obtained by immunizing an animal other than human with a protein of amino acid sequence of SEQ ID NO.1 of the sequence listing.

The antibody used in the present invention is a polyclonal antibody.

In the present invention, the aforementioned polyclonal antibody may be used as an immobilized antibody immobilized on an insoluble carrier such as solid carrier, or may be used as a labeled antibody labeled with a labeling substance. An immobilized polyclonal antibody is an antibody in a state of being carried on an insoluble carrier by physical adsorption, chemical bond or the like. Such an immobilized polyclonal antibody may be used for detection, quantitative determination, separation or purification of antigen (i.e., a protein of amino acid sequence of SEQ ID NO.1 of the sequence listing) contained in a sample (such as hair, follicles or an extract thereof). Examples of the insoluble carrier which can be used for immobilization of the polyclonal antibody include (1) a container having an inner volume such as plate, test tube or tube, or beads, ball, filter, membrane and the like, each of which are made of water-insoluble substance such as plastics including polystyrene resin, polycarbonate resin, silicone resin or Nylon resin, or glass; (2) an insoluble carrier used for affinity chromatography, such as cellulose based carrier, agarose based carrier, polyacrylamide based carrier, dextran based carrier, polystyrene based carrier, polyvinyl alcohol based carrier, polyamino acid based carrier or porous silica based carrier.

A labeled polyclonal antibody means an antibody labeled with a labeling substance, and such a labeled antibody may be used for detection or quantitative determination of antigen (i.e., a protein of amino acid sequence of SEQ ID NO.1 of the sequence listing) contained in a sample (such as hair, follicles or an extract thereof). There is no particular limitation for the labeling substance used in the present invention, so far as its existence can be detected by bonding to an antibody by means of physical bonding, chemical bonding or the like. Examples of the labeling substance are enzyme, fluorescent substance, chemiluminescent substance, biotin, avidin or radioactive isotope. Specific examples include enzyme such as peroxidase, alkaline phosphatase, β-D-galactosidase, glucose oxidase, glucose-6-phosphate dehydrogenase, alcohol dehydrogenase, malic acid dehydrogenase, penicillinase, catalase, apoglucose oxidase, urease, luciferase or acetylcholine esterase; fluorescent substance such as fluorescein isothiocyanate, phycobilic protein, rare earth metal chelate, dansyl chloride or tetramethylol rhodamine isothiocyanate; radioisotope such as ³H, ¹⁴C, ¹²⁵I or ¹³¹I; biotin; avidin; and chemiluminescent substance. With regard to a method for bonding a labeling substance to a polyclonal antibody, known methods such as a glutaraldehyde method, a maleimide method, a pyridyl disulfide method and a periodic acid method may be used.

Here, each of the radioisotope and fluorescent substance is able to generate a detectable signal by itself, while each of enzyme, chemiluminescent substance, biotin and avidin is unable to generate a detectable signal by itself and, therefore, a detectable signal is generated as a result of reaction with one or more other substance(s). For example, in the case of an enzyme, at least a substrate is necessary and, depending upon a method for measuring the enzymatic activity (colorimetric method, fluorescent method, bioluminescent method or chemiluminescent method), various substrates are used. In the case of biotin, it is usual that at least avidin or enzyme- bound avidin is reacted therewith. If necessary, various coloring substances may be used depending upon the said substrate.

The polyclonal antibody which recognizes a protein of amino acid sequence of SEQ ID NO.1 of the sequence listing can be produced by a conventional method.

For example, a mammal is immunized with a protein having an amino acid sequence of SEQ ID NO.1 of the sequence listing or a sample containing said protein (for example, proteins extracted from hair collected from the skin of the growth period and/or follicles of whiskers of the growth period) as an immunogen, and blood is collected from the mammal. Then, antibodies are separated and purified from the collected blood, and thereby a polyclonal antibody can be obtained. For example, mammals such as mouse, hamster, guinea pig, chicken, rat, rabbit, dog, goat, sheep and bovine can be immunized. The method of immunization is known to a skilled person in the art, and can be performed, for example, by administering an antigen once or more. For example, an antigen may be administered two or three times at an interval of 7 to 30 days. For example, a dose of about 0.05 to 2mg of antigen can be used per administration. The route of administration is not specifically limited, and can be appropriately selected from subcutaneous, intracutaneous, intraperitoneal, intravenous, and intramuscular administrations. Administration by intravenous, intraperitoneal, or subcutaneous injection is preferred. Antigens may be used by dissolving in an appropriate buffer, for example an appropriate buffer containing a general adjuvant, such as complete Freund's adjuvant or aluminum hydroxide. There may be cases where the above adjuvant may not be used depending on administration routes, conditions or the like.

An immunized mammal is reared for certain period, a serum of the mammal is sampled, and then the antibody titer is measured. When the antibody titer starts to elevate, booster immunization is performed by using, for example, 10 µg to 1000µg of the antigen. Blood is collected from an immunized mammal 1 to 2 months after the final administration. The collected blood is separated and purified by standard techniques including centrifugation, precipitation using ammonium sulfate or polyethylene glycol, and chromatography such as gel filtration chromatography, ion exchange chromatography and affinity chromatography. As a result, polyclonal antibodies which recognize the protein of the present invention can be obtained as a polyclonal anti-serum. The complement system can be inactivated by treating the anti-serum, for example, at 56°C for 30 minutes.

The method for the evaluation of hair growth promoting activity according to the present invention may be any method so far as it is an assay using the aforementioned polyclonal antibody or, in other words, an immunoassay. Examples thereof include western blotting, enzyme-linked immunosorbent assay (ELISA), fluoroimmunoassay, radioimmunoassay (RIA), luminoimmunoassay, immunoenzymatic assay, immunofluorescence assay, immunoturbidimetry, latex agglutination reaction, latex turbidimetry, erythrocyte agglutination reaction and particle agglutination reaction.

There is no particular limitation for the type of the test substance which is subjected to the method for the evaluation of the present invention, and the test substance may be either oligopeptides or low-molecular organic compounds. For example, there may be used an oligopeptide having a partial amino acid sequence of epimorphin which has been known to have a hair growth promoting activity.

When the method for the evaluation of hair growth promoting activity according to the present invention is carried out by means of an immunoassay using labeled polyclonal antibody such as enzyme-linked immunosorbent assay, fluoroimmunoassay, radioimmunoassay or luminoimmunoassay, it is also possible to carry out the assay by a sandwich method or a competition method. In the case of a sandwich method, at least one of solid phase antibody and labeled antibody is the antibody of the present invention.

With regard to the solid phase carrier, there may be used the above-mentioned carriers which are described in the present specification as specific examples for the insoluble carrier in relation to the immobilized polyclonal antibody. Also with regard to the labeled substance, there may be used the above-mentioned substances which are described in the present specification in relation to the labeled antibody.

A method for the measurement may be carried out by a known method ("Enzyme-Linked Immunosorbent Assay", Supplementary Issue No. 31 of Tampakushitsu, Kakusan, Koso, edited by Tsunehiro Kitagawa, et al., published by Kyoritsu Shuppan, 1987).

For example, a solid phase polyclonal antibody is reacted with a sample and a labeled polyclonal antibody at the same time, or a solid phase polyclonal antibody is reacted with a sample, and after being washed, it is reacted with a labeled polyclonal antibody, thereby forming a complex of solid phase antibody-antigen-labeled antibody. Then, unbound labeled polyclonal antibody is separated by washing, and the amount of the antigen in the sample can be measured from the amount of the bound labeled polyclonal antibody. Specifically, in the case of the enzyme-linked immunosorbent assay (ELISA), the labeled enzyme is reacted with a substrate under an optimum condition and the amount of the reaction product is measured by, for example, an optical method. In the case of the fluoroimmunoassay, intensity of fluorescence by a fluorescent substance labeling is measured, and in the .case of the radioimmunoassay, radiation dose by a radioactive substance labeling is measured. In the case of the luminoimmunoassay, amount of luminescence in the luminous reaction system is measured.

In the method for the detection and/or the quantitative determination according to the present invention, when the production of an immune complex aggregate by immunoturbidimetry, latex agglutination reaction, latex turbidimetry, erythrocyte agglutination reaction, particle agglutination reaction or the like is measured by measuring its transmitting light or scattering light by an optical method, or is measured visually, it is possible to use phosphate buffer, glycin buffer, Tris buffer, Good buffer or the like as a solvent, and a reaction promoting agent such as polyethylene glycol or a non-specific reaction suppressing agent may be contained therein.

When a polyclonal antibody is used by sensitizing it to a solid phase carrier, there may be used particles made of the material such as polystyrene, styrene-butadiene copolymer, (meth)acrylate polymer, latex, gelatin, liposome, microcapsule, erythrocyte, silica, alumina, carbon black, metal compound, metal, ceramics or magnetic substance as a solid phase carrier.

The methods for the sensitization include known method such as physical adsorption, chemical bonding or a combination thereof. A method for the measurement may be carried out by a known method. For example, in the case of the measurement by an optical method, the sample is reacted with the polyclonal antibody, or the sample is reacted with the polyclonal antibody which was sensitized with a solid phase carrier. Then, the transmitting light or the scattering light is measured by an end-point method or a rate method.

When the measurement is carried out visually, the sample is reacted with the polyclonal antibody sensitized with a solid phase carrier in a container such as a plate or a microtiter plate, and the state of agglutination is judged visually. Instead of measuring visually, the measurement may be carried out by an instrument such as a microplate reader.

### Examples

The present invention will be explained more specifically by the following Examples. However, the scope of the invention is not limited to these examples.

### Example 1: Protein expressed at a high level in anagen stage of hair cycle

### (1) Preparation of expression library

Using TRIZOL(GIBCO 15596-018) in accordance with a manual attached therewith, total RNA was prepared from the back of a C57BL mouse, the hair cycle of which was in anagen (35 days after birth). Using Quick Prep^{™} micro mRNA purification kit manufactured by Amersham Pharmacia, mRNA (20 µg) was prepared from the total RNA. Thereafter, cDNA was synthesized from 5 µg of the mRNA with random primers, employing Time Saver cDNA Synthesis kit 27-9262-01 manufactured by Amersham Pharmacia. Due to the use of adaptors attached with the aforementioned kit, the synthesized cDNA had *Eco*RI and *Not*I sites at both termini thereof. The obtained cDNA was inserted into λExCell *Eco*RI/CIP (27-5011-01) manufactured by Amersham Pharmacia, so as to produce an expression library.

*Escherichia coli* MN522 was infected with the thus produced expression library, and the obtained plaques were dispersed on an LB plate at a concentration of 20,000 plaques/plate. The LB plate was then immersed in a 10 mM IPTG solution, followed by air drying. Thereafter, the LB plate was covered with nitrocellulose, and it was left at rest overnight.

### (2) Preparation of a monoclonal antibody which is specific for new follicle

Hairs were cut off from the skin of B57BL mouse of growing stage (48 to 50 days), and were incubated overnight at 37°C in PBS containing 8M urea, 2% SDS and 100mM DTT, thereby the protein was extracted. Further, whisker follicles of B57BL mouse (where hair ball portion is stained with pigment; growing stage (anagen)) were collected with a stereoscopic microscope, and were homogenized in PBS. The above 2 samples (0.5mg of protein weight) were mixed, and mixed with the same amount of the complete adjuvant to prepare micelle.

The above-obtained micelle (0.2mg) was subcutaneously (3 sites) administered to a rat (Wister) for immunization. After the first immunization, the booster was performed in the same way as in the above. After 2 weeks, the second booster was performed in the same way as in the above. On the third day after the second booster, a spleen was removed from the immunized rat, and the blood cells were collected by mesh. Antibody producing cells are contained in these blood cells. All amount of the above-collected blood cells were mixed with mouse myeloma P3U1 using polyethyleneglyco 1500, and were suspended in Dulbecco/Hum F12 mixed medium (10⁷ cells/ml). 100µl of the culture were inoculated in each well of 96-well plate. On the next day, the same amount (100µl) of HAT medium (Sigma) was added to each well. After 2 days, 150µl of the medium was removed under aspiration from each well, and 150µl of the fresh medium was added to each well. The 96-well plate was placed in CO₂ incubator at 37°C.

The follicles of the growing whisker of B57BL mouse were dissolved in 8M urea by ultrasonic treatment. A nitrocellulose membrane was immersed in this solution for 5 minutes, and was washed well with PBS. Biorad dot blotter equipped with the above membrane was used to perform the first screening of the hybridoma supernatant which was recovered from each well of the above 96-well plate. First, the above prepared nitrocellulose membrane which was equipped in Biorad dot blotter was blocked with Tris buffer containing 5% skim milk (TBST), and then 100µl of the hybridoma supernatant was added to each well. After incubation for 1 hour, the wells were washed with Tris buffer, and the second antibody, horseradish peroxidase labeled anti-rat IgG (1µg/ml in TBST), was added. ECL agent (AmershamPharmacia), which is a coloring substrate, was added, and 50 antibodies in total which reacted with the growing whisker follicle were selected by detection of coloring (first screening).

Among the 50 antibodies selected in the above first screening, the antibodies which specifically reacted with the frozen segment (10µm) of the growing whisker follicle were selected (second screening). Specifically, the frozen segment of the growing whisker follicle was placed on a slide glass, and the hybridoma supernatant selected in the first screening was added thereto, and the coloring was developed with the second antibody. More specifically, the frozen segment of the growing whisker follicle which was prepared by Cryosdat (Bright) was treated with methanol at -20°C, and was blocked with TBST for 1 hour, and then was reacted with the hybridoma supernatant for 1 hour. The sample was washed with Tris buffer, and was reacted with FITC-labeled anti-rat IgG (100µg/ml in TBST). The sample was washed with Tris buffer, and was covered with a cover glass. The observation was carried out under fluorescent microscope.

As a result of the second screening, 8 antibodies were selected. These antibodies do not react with epidermis, and specifically react with follicle. These 8 antibodies were cloned by the limiting dilution.

The reactivity of these 8 antibodies was examined by Western Blotting using growing whisker follicle or resting whisker follicle as a sample, and using slide samples of the skins having follicles derived from 14 day fetal mouse. As a result, mAb27 was obtained as a monoclonal antibody which specifically reacted with growing whisker follicle and plastic follicle (new follicle), and did not react with resting whisker follicle. The hybridoma which produced monoclonal antibody mAb27 was deposited with Patent and Bio-Resource Center of National Institute of Advanced Industrial Science and Technology (Chuo-6, 1-1, Higashi 1-chome, Tsukuba-shi, Ibaraki-ken, Japan) on November 2, 2001 under the deposit number of FERM P-18578, and this deposit was transferred to the International deposit on July 22, 2002 under the deposit number of FERM BP-8121.

### (3) Immunoscreening

The *Escherichia coli* expression library produced in (1) above was subjected to immunoscreening using the monoclonal antibody mAb27 produced in (2) above. Specifically, a library of 300,000 clones was developed (20,000 plaques/plate), and nitrocellulose into which IPTG had been infiltrated was then made to closely come into contact with the plaques, so that expressed gene products were transcribed onto the nitrocellulose. The nitrocellulose was blocked with 1% skim milk/TBS (STBS). Thereafter, it was incubated with 10 µg/ml mAb27 and HRP-labeled anti-rat IgG (Amersham), followed by detection with ECL (Amersham).

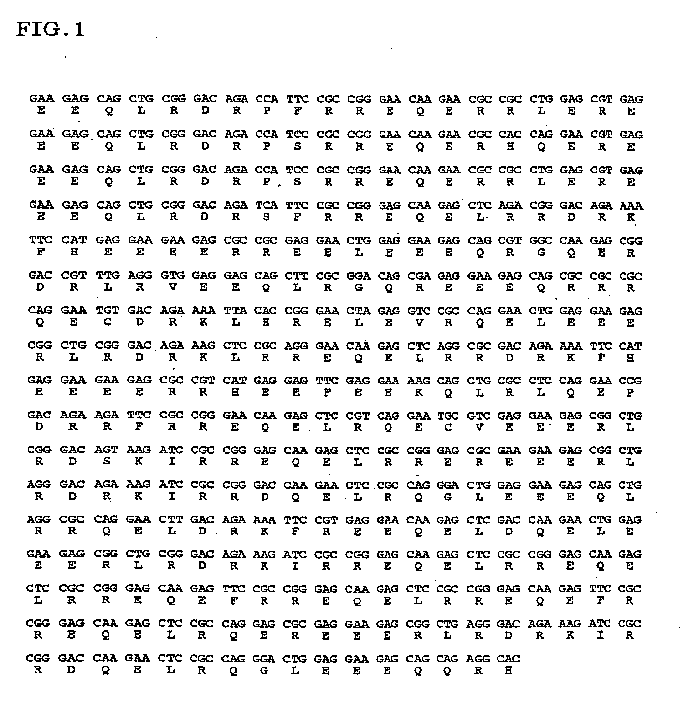

The thus obtained 6 positive clones were recovered, and their nucleotide sequences were determined. As a result, it was found that all 6 clones shared a common sequence (Figure 1).

### Example 2: Database search

The database (NCIB (GeneBank)) was searched for the common sequence (Figure 1) identified in Example 1. As a result, a registration No. XM_177952 (*Mus musculus*, similar to Trichohyalin) was found. The amino acid sequence of registration No. XM_177952 is shown in SEQ ID NO: 1, and the nucleotide sequence thereof is shown in SEQ ID NO: 2. The amino acid sequence shown in Figure 1 corresponds to a portion at positions 800 to 1135 of the amino acid sequence shown in SEQ ID NO: 1. However, Leu at position 1133 of the amino acid sequence shown in SEQ ID NO: 1 is substituted by Gln in Figure 1, and Arg at position 1135 of the amino acid sequence shown in SEQ ID NO: 1 is substituted by His in Figure 1.

### Example 3: Production of a recombinant protein

### (1) Preparation of full-length cDNA by PCR

A cDNA library was produced from the mRNA of the skin in the growth period, using oligo dT primers and reverse transcriptase. Using the obtained cDNA library as a template, PCR was carried out with two types of primers (5'-atgtctccacttataagaagcattgtagat-3' (SEQ ID NO: 3) and 5'-ttaagggcggtattgagacctctgctcctg-3' (SEQ ID NO: 4)), so that full-length cDNA was cloned. LA Taq^{™} kit (Takara) was used in the PCR reaction, and the PCR conditions consisted of: 94°C, 1 minute; 94°C, 30 seconds; 60°C, 30 seconds; and 72°C, 2 minutes, so that the cDNA of interest was amplified by 30 cycles.

The obtained clone contained full-length cDNA encoding the amino acid sequence shown in SEQ ID NO: 1.

### (2) Transformation

The PCR product obtained in (1) above was inserted into a pTarget vector (Promega), so as to construct a recombinant expression vector. Thereafter, COS-1 cells as host cells were transfected with the recombinant expression vector. Transfection was carried out using Lipofectamine (GIBCO) in accordance with the manual attached therewith. The COS-1 cells were cultured in a Dalbecco's Ham's F12 medium to which 10% FCS had been added.

### (3) Extraction of recombinant protein

### (a) Preparation of affinity column

Afigel 10 (Biorad) was adjusted to 4°C, so as to make the gel homogenous. The gel was then placed in a Buchner's funnel, followed by filtration. Thereafter, the gel was washed with 3 times its volume of deionized water at 4°C. After washing, the gel was placed in a flask, and an antibody solution obtained by dissolving a monoclonal antibody mAb27 in PBS (wherein 0.5 ml of an antibody solution was used per 1 ml of the gel) was added into the flask. The mixture was fully stirred and suspended. While slowly stirring the suspension with a stirrer, it was reacted at room temperature for 1 hour. The supernatant was eliminated by centrifugation, and the reaction solution was then adjusted with PBS to 10 ml. 0.1 ml of 1M glycine ethyl ester (pH 8) per ml of the gel was added, and the mixture was allowed to react for 1 hour. After completion of the reaction, the gel was packed into an Ecopac column (available up to 20 ml, Biorad), and it was then washed with distilled water until no reaction products were detected by OD₂₆₀. Finally, it was washed with an eluent (10 mM Tris-HCl, 1 mM MgCl₂, 1 mM EDTA, protein inhibitor cocktail (product name: CompleteMini; Roche), 0.15 M NaCl, and 0.5% TritonX-100; adjusted to pH 3.0 with HCl).

### (b) Purification of antigen

The transformed COS-1 cells produced in Example 2, in which full-length cDNA was expressed, were recovered on the third day after the transfection, and washed with PBS. Thereafter, the cells were added to 100 ml of a mixed solution of 10 mM Tris-HCl (pH 7.5), 1 mM MgCl₂, 1 mM EDTA, and protein inhibitor cocktail (product name: CompleteMini; Roche). Then, they were fully crushed with a homogenizer. Subsequently, NaCl was added thereto to a final concentration of 0.15 M, and TritonX-100 was then added thereto to a final concentration of 0.5%. The obtained mixture was stirred at 4°C for 3 hours with a stirrer. The mixture was then centrifuged at 25,000 rpm for 10 minutes, and the supernatant was recovered. Thereafter, a protein was extracted from the cells. 100 ml of the obtained extract was subjected to the affinity column of (1) above.

Washing was carried out with a washing solution (10 mM Tris-HCl (pH 7.5), 1 mM MgCl₂, 1 mM EDTA, protein inhibitor cocktail (product name: CompleteMini; Roche), 0.15 NaCl, and 0.5% TritonX-100). Thereafter, an eluent (a solution obtained by adjusting the above washing solution to pH 3.0 with HCl) was added to the column. The eluant was recovered in amounts of 2 ml each. A protein-containing fraction was then recovered by absorption at OD 280 nm.

### (c) Measurement of molecular weight of purified antigen by electrophoresis

The protein-containing fraction recovered in (b) above was electrophoresed by SDS-PAGE, and then, Western blotting was carried out using a monoclonal antibody mAb27. As a result, a band was detected at the position of 220 kDa. In addition, the gel which was obtained by the electrophoresis of the protein-containing fraction recovered in (b) above by SDS-PAGE, was stained with Coomassie blue, and the band of 220 kDa was cut out, so as to prepare a purified antigen.

### Example 4: Production of polyclonal antibody which recognizes a protein of amino acid sequence shown in SEQ ID NO: 1 of the sequence listing

The purified antigen prepared in Example 3 (that is, the excised gel containing the protein of the amino acid sequence shown in SEQ ID NO: 1) was recovered and placed in 20% ethanol, and then left overnight. Thereafter, the gel was recovered. A sample containing approximately 100 µg or less of the protein was mixed with Titer Max Gold (an adjuvant from CytRX Corp.) to produce an emulsion. A rat was immunized with the emulsion for 2 months (3 times in total). Two months after the primary immunization, the blood was collected from the rat, and the serum was then prepared by common methods, so as to obtain a polyclonal antibody which recognizes the protein of the amino acid sequence shown in SEQ ID NO: 1.

### Example 5: Evaluation of hair growth-promoting activity, using polyclonal antibody which recognizes a protein of amino acid sequence shown in SEQ ID NO: 1 of the sequence listing

Differentiation of human keratinocytes was evaluated. Human keratinocytes (which are skin cells containing epidermal keratinocytes and hair follicle-derived cells) were purchased from Sanko Junyaku. A peptide ssb7 (which was obtained by biotinating the N-terminus of Ser-Ile-Glu-Gln-Ser-Cys-Asp-Gln-Asp-Glu (SEQ ID NO: 5) (NHS-Biotin, Pierce) and then subjecting it to S-S crosslinking) was added to a culture solution attached with the purchased cells, such that the concentration became 20 µM. It was cultured on a 96-well plate for 1 week. Thereafter, cells were recovered from the wells. The cells were placed in 100 µl of an SDS sample buffer (0.02 g/ml SDS, 0.2 g/ml glycerol, pH 6.8), and they were then dissolved in the buffer using an ultrasonic disintegrator. A control (human keratinocytes that were cultured for 1 week without adding ssb7) was treated in the same manner as described above. A solution obtained by the above treatment was electrophoresed (35 mA, 1.5 hours) by SDS-PAGE (4% to 20% acrylamide), and the resultant product was transferred to a PVDF membrane, followed by incubation in a Tris buffer solution (TBST) containing 5% skim milk for 1 hour. It was allowed to react for 1 hour with a polyclonal antibody (obtained by diluting serum to 1/100) against a protein having an amino acid sequence shown in Figure 1, which was a partial sequence of the amino acid sequence shown in SEQ ID NO: 1. Thereafter, the reaction product was fully washed with TBS. It was then allowed to react with a secondary antibody obtained by diluting peroxidase-labeled anti-rat IgG (Amersham) with TBST to 1/1,000. Thereafter, the resultant product was fully washed, and the level of the reactivity of the polyclonal antibody was examined using an ECL kit (Amersham).

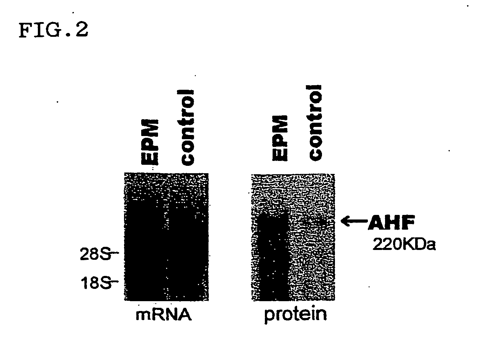

The obtained results are shown in the right figure of Figure 2.

In the right figure of Figure 2, the right lane represents the results of the control, and the left lane represents the results obtained by addition of EPM (ssb7), which was a hair growth tonic. AHF in the figure represents a protein of the amino acid sequence shown in SEQ ID NO: 1 of the sequence listing. As is clear from the results in Figure 2, in the sample to which EPM (ssb7) as a hair growth tonic had been added, a band was detected, which was expressed more highly than that of the control. This result reflects that the expression level of the protein of the amino acid sequence shown in SEQ ID NO: 1 of the sequence listing was increased.

The expression of the protein of the amino acid sequence shown in SEQ ID NO: 1 was analyzed at the mRNA level by Northern blotting in the same manner as described above. Specifically, first, 10 µg of total RNA prepared from the back skin of a rat was electrophoresed in agarose gel, and the resultant sample was transferred to a nylon membrane (HibondN+, Amersham). Subsequently, a label was introduced into the translation region of AHF cDNA using DIG (manufactured by Roche). Using this as a probe, AHF mRNA that had been transferred to the aforementioned nylon membrane was detected.

The obtained results are shown in the left figure of Figure 2. As in the case of the right figure of Figure 2, in the sample to which EPM (ssb7) as a hair growth tonic had been added, a band that was stronger than that of the control was detected.

### Industrial Applicability

According to the method of the present invention, the hair growth promoting activity of a test substance can be efficiently evaluated.

## Claims

1. A method for the evaluation of hair growth promoting activity which comprises the steps of:
incubating skin tissue pieces derived from living organism in the presence of a substance to be tested;
recovering said skin tissue pieces, and reacting them with a polyclonal antibody which recognizes a protein of amino acid sequence SEQ ID NO: 1 of the sequence listing; and
detecting or measuring said antibody which has reacted with the skin tissue pieces.

## Patentansprüche

1. Ein Verfahren zur Beurteilung der Haarwachstum fördernden Wirkung umfassend die Schritte:
Inkubieren von aus lebenden Organismen stammenden Hautgewebeproben in Gegenwart einer zu testenden Substanz; Wiedergewinnen dieser Hautgewebeproben und reagieren lassen derselben mit einem polyklonalen Antikörper, der ein Protein mit der Aminosäuresequenz SEQ ID NO: 1 gemäß der Sequenzliste erkennt; und
Nachweisen oder Messen des Antikörpers, der mit den Hautgewebeproben reagiert hat.

## Revendications

1. Procédé d'évaluation d'une activité favorisant la pousse des cheveux qui comprend les étapes suivantes:
l'incubation de fragments de tissus de la peau provenant d'un organisme vivant en présence d'une substance à tester ;
la récupération desdits fragments de tissus de la peau, et la mise en réaction de ceux-ci avec un anticorps polyclonal qui reconnaît une protéine de la séquence d'acides aminés SEQ ID NO :1 du listage des séquences ; et
la détection ou la mesure dudit anticorps qui avait réagi avec les fragments de tissus de la peau.


### SEQUENCE LISTING

<110> Sumitomo Electric
<120> A METHOD FOR THE EVALUATION OF HAIR GROWTH PROMOTING ACTIVITY
<130> P21444EP
<140> EP 04 700 512.9
   <141> 2004-01-07
<150> JP 001891/2003
   <151> 2003-01-08
<160> 5
<170> PatentIn Ver. 2.1
<210> 1
   <211> 1439
   <212> PRT
   <213> Mus musculus (house mouse)
<400> 1
   Met Ser Pro Leu Ile Arg Ser Ile Val Asp Ile Thr Glu Val Phe Asn
    1               5                  10                  15
   Gln Tyr Ala Ser Gln Ser Cys Asp Gly Ala Ser Leu Ser Lys Lys Asp
               20                  25                  30
   Leu Lys Asn Leu Leu Glu Arg Glu Leu Gly Asp Val Leu Gln Arg Pro
           35                  40                  45
   His Asp Pro Glu Thr Ile Asp Leu Thr Leu Glu Leu Leu Asp Arg Asp
       50                  55                  60
   Cys Asn Gly Arg Val Asp Phe Asn Glu Phe Leu Leu Phe Leu Phe Lys
   65                  70                  75                  80
   Ile Ala Gln Ala Cys Tyr Tyr Ala Leu Asp Gln Ala Ala Glu Leu Gly
                   85                  90                  95
   Glu Lys Arg Ala Leu Pro Asn Glu Lys Arg Asn Leu Ser Gln Asp Arg
               100                 105                 110
   Arg Gln Glu Asp Gln Arg Arg Phe Glu Pro Arg Ser Arg Gln Leu Asp
           115                 120                 125
   Glu Glu Pro Gly Arg Arg Ser Trp Gln Lys Arg Arg Glu Gln Glu Glu
   130 135 140
   Arg Ala Glu Glu Gln Arg Leu Glu Gln Arg Tyr Arg Gln His Arg Asp
   145 150 155 160
   Glu Glu Gln Arg Leu Gln Arg Arg Glu Leu Gln Glu Leu Glu Glu Arg
   165 170 175
   Leu Ala Glu Lys Glu Pro Leu Gly Trp Ser Lys Gly Arg Asp Ala Glu
   180 185 190
   Glu Phe Ser Glu Val Glu Glu Gln Gln Arg Gln Glu Arg Gln Glu Leu
   195 200 205
   Lys Gly Lys Gly Gln Thr Glu Glu Arg Arg Leu Gln Lys Arg Arg Gln
   210 215 220
   Glu Glu Leu Arg Glu Pro Leu Leu Arg Arg Asp Leu Glu Leu Arg Arg
   225 230 235 240
   Glu Gln Glu Leu Arg Arg Glu Gln Glu Leu Arg Gln Glu Gln Arg Arg
   245 250 255
   Glu Gln Glu Leu Arg Arg Glu Gln Glu Leu Arg Gln Glu Leu Arg Arg
   260 265 270
   Glu Gln Glu Leu Asn Arg Arg Gln Glu Leu Arg Arg Glu Gln Glu Leu
   275 280 285
   Arg Arg Glu Gln Glu Leu Arg Gln Glu Leu Arg Arg Glu Gln Glu Leu
   290 295 300
   Arg Arg Glu Gln Glu Leu Arg Gln Glu Leu Arg Arg Glu Gln Glu Leu
   305 310 315 320
   Arg Arg Glu Gln Glu Leu Arg Gln Glu Leu Arg Arg Glu Gln Glu Leu
   325 330 335
   Arg Arg Glu Gln Glu Leu Arg Gln Glu Leu Arg Arg Glu Gln Glu Leu
   340 345 350
   Arg Arg Glu Gln Glu Leu Arg Gln Glu Leu Ala Glu Glu Asp Glu Leu
   355 360 365
   Thr Arg Ile Arg Glu Pro Asp Glu Ser Ile Thr Gln Arg Trp Gln Trp
   370 375 380
   Gln Leu Glu Asn Glu Ala Asp Ala Arg Gln Asn Lys Val Tyr Ser Arg
   385 390 395 400
   Pro Ser Arg Gln Glu Gln Arg Leu Arg Gln Glu Leu Gly Glu Arg Gln
   405 410 415
   Leu Arg Glu Gln Glu Glu Gln Arg Arg Asp Leu Gln Gln Glu Arg Pro
   420 425 430
   Ala Glu Glu Ala Arg Gln Arg Asn Gln Trp Glu Arg Pro Gln Arg Ala
   435 440 445
   Glu Glu Arg Leu Glu Gln Glu Gln Arg Phe Arg Asp Arg Glu Glu Gln
   450 455 460
   Arg Phe Arg Glu Glu Lys Leu Gln Arg Ala Glu Leu Gln Asp Ser Leu
   465 470 475 480
   Leu Asp Glu Glu Gln Arg Arg Leu Gln Glu Glu Arg Arg Glu Pro Asn
   485 490 495
   Arg Ser Arg Gln Leu Arg Glu Glu Ser Gln Arg Arg Arg Thr Leu Tyr
   500 505 510
   Ala Lys Pro Ser Gln Arg Gln Gln Arg Arg Arg Leu Gln Gln Glu Arg
   515 520 525
   Gln Tyr Gln Glu Glu Asp Leu Gln Arg Leu Arg Asp Glu Asp Gln Arg
   530 535 540
   Arg Asp Leu Lys Trp Gln Trp Gln Pro Arg Lys Glu Asn Glu Val Arg
   545 550 555 560
   Ser Asn Arg Leu Phe Thr Lys Arg Arg Gly Asp Glu Glu Pro Ile Gln
   565 570 575
   Gln Leu Glu Asp Ser Gln Glu Arg Glu Arg Arg Gln Asp Arg Arg Pro
   580 585 590
   Leu Gln Asp Glu Glu Glu Glu Lys Arg Glu Leu Glu Gln Glu Arg Arg
   595 600 605
   Arg Arg Gln Gln Arg Asp Arg Gln Ile Leu Glu Glu Glu Gln Phe Gln
   610 615 620
   Arg Glu His Gln Arg Glu Ala Arg Arg Arg Asp Glu Thr Phe Gln Glu
   625 630 635 640
   Glu Glu Gln Leu Gln Gly Glu Ser Arg Arg Arg Gln Gln Glu Arg Glu
   645 650 655
   Gly Lys Phe Leu Glu Glu Glu Arg Gln Leu Arg Thr Glu Arg Glu Glu
   660 665 670
   Gln Arg Arg Arg Gln Glu Gln Glu Arg Glu Phe Gln Glu Glu Glu Glu
   675 680 685
   His Leu Gln Glu Arg Glu Lys Glu Leu Arg Gln Glu Cys Asp Arg Lys
   690 695 700
   Ser Arg Glu Gln Glu Arg Arg Gln Gln Arg Glu Glu Glu Gln Leu Arg
   705 710 715 720
   Arg Gln Glu Arg Asp Gln Arg Phe Arg Arg Glu Gln Glu Arg His Leu
   725 730 735
   Glu Arg Glu Glu Glu Gln Leu Arg Asp Arg Pro Ser Arg Arg Glu Gln
   740 745 750
   Glu Arg His Gln Glu Arg Glu Glu Glu Gln Leu Arg Asp Arg Pro Ser
   755 760 765
   Arg Arg Glu Gln Glu Arg His Gln Glu Arg Glu Glu Glu Gln Leu Arg
   770 775 780
   Asp Arg Pro Ser Arg Arg Glu Gln Glu Arg His Gln Glu Arg Glu Glu
   785 790 795 800
   Glu Gln Leu Arg Asp Arg Pro Phe Arg Arg Glu Gln Glu Arg Arg Leu
   805 810 815
   Glu Arg Glu Glu Glu Gln Leu Arg Asp Arg Pro Ser Arg Arg Glu Gln
   820 825 830
   Glu Arg His Gln Glu Arg Glu Glu Glu Gln Leu Arg Asp Arg Pro Ser
   835 840 845
   Arg Arg Glu Gln Glu Arg Arg Leu Glu Arg Glu Glu Glu Gln Leu Arg
   850 855 860
   Asp Arg Ser Phe Arg Arg Glu Gln Glu Leu Arg Arg Asp Arg Lys Phe
   865 870 875 880
   His Glu Glu Glu Glu Arg Arg Glu Glu Leu Glu Glu Glu Gln Arg Gly
   885 890 895
   Gln Glu Arg Asp Arg Leu Arg Val Glu Glu Gln Leu Arg Gly Gln Arg
   900 905 910
   Glu Glu Glu Gln Arg Arg Arg Gln Glu Cys Asp Arg Lys Leu His Arg
   915 920 925
   Glu Leu Glu Val Arg Gln Glu Leu Glu Glu Glu Arg Leu Arg Asp Arg
   930 935 940
   Lys Leu Arg Arg Glu Gln Glu Leu Arg Arg Asp Arg Lys Phe His Glu
   945 950 955 960
   Glu Glu Glu Arg Arg His Glu Glu Phe Glu Glu Lys Gln Leu Arg Leu
   965 970 975
   Gln Glu Pro Asp Arg Arg Phe Arg Arg Glu Gln Glu Leu Arg Gln Glu
   980 985 990
   Cys Val Glu Glu Glu Arg Leu Arg Asp Ser Lys Ile Arg Arg Glu Gln
   995 1000 1005
   Glu Leu Arg Arg Glu Arg Glu Glu Glu Arg Leu Arg Asp Arg Lys Ile
   1010 1015 1020
   Arg Arg Asp Gln Glu Leu Arg Gln Gly Leu Glu Glu Glu Gln Leu Arg
   1025 1030 1035 1040
   Arg Gln Glu Leu Asp Arg Lys Phe Arg Glu Glu Gln Glu Leu Asp Gln
   1045 1050 1055
   Glu Leu Glu Glu Glu Arg Leu Arg Asp Arg Lys Ile Arg Arg Glu Gln
   1060 1065 1070
   Glu Leu Arg Arg Glu Gln Glu Leu Arg Arg Glu Gln Glu Phe Arg Arg
   1075 1080 1085
   Glu Gln Glu Leu Arg Arg Glu Gln Glu Phe Arg Arg Glu Gln Glu Leu
   1090 1095 1100
   Arg Gln Glu Arg Glu Glu Glu Arg Leu Arg Asp Arg Lys Ile Arg Arg
   1105 1110 1115 1120
   Asp Gln Glu Leu Arg Gln Gly Leu Glu Glu Glu Gln Leu Arg Arg Gln
   1125 1130 1135
   Glu Arg Asp Arg Lys Phe Arg Glu Glu Gln Glu Leu Gly Gln Glu Leu
   1140 1145 1150
   Glu Glu Glu Arg Leu Arg Asp Arg Lys Ile Arg Arg Glu Gln Glu Leu
   1155 1160 1165
   Arg Arg Glu Arg Glu Gln Glu Gln Arg Arg Arg Leu Glu Arg Glu Glu
   1170 1175 1180
   Glu Gln Gln Arg Leu His Glu Arg Glu Glu Glu Gln Arg Arg Arg Gln
   1185 1190 1195 1200
   Glu Arg Glu Gln Glu Gln Gln Arg Cys Leu Glu Arg Glu Glu Glu Gln
   1205 1210 1215
   Phe Arg Phe Glu Glu Gln Gln Arg Arg Arg Gln Glu Arg Glu Gln Gln
   1220 1225 1230
   Leu Arg Gln Glu Arg Asp Arg Arg Val Leu Glu Glu Glu Glu Leu Arg
   1235 1240 1245
   Gln Glu Arg Glu Glu Leu Leu His Arg Gln Val Gly Gly Arg Lys Phe
   1250 1255 1260
   Arg Glu Glu Glu Arg Leu Arg Leu Glu Arg Glu Glu Gln Gln Arg Arg
   1265 1270 1275 1280
   Leu Gln Glu Arg Asp Asn Arg Arg Phe Arg Glu Glu Val Glu Leu Arg
   1285 1290 1295
   Gln Glu Arg Glu Gly Gln Gln Leu Arg Gln Glu Arg Asp Arg Lys Phe
   1300 1305 1310
   Arg Glu Val Glu Glu Leu Arg Gln Glu Glu Gln Arg Arg Arg Gln Glu
   1315 1320 1325
   Arg Asp Arg Lys Phe Arg Glu Glu Lys His Pro Arg Glu Glu Arg Glu
   1330 1335 1340
   Glu Gln Gln Leu Arg Arg Glu Lys Arg Asp Gly Gln Tyr Leu Ala Glu
   1345 1350 1355 1360
   Glu Gln Phe Ala Arg Asp Thr Ile Arg Arg Gln Glu Gln Glu Leu Arg
   1365 1370 1375
   Gln Glu Glu Glu Gln Arg Arg Arg Gln Glu Arg Glu Arg Lys Phe Gln
   1380 1385 1390
   Glu Glu Gln Ile Arg Arg Arg Gln Glu Glu Gln Arg Arg Arg Gln Ile
   1395 1400 1405
   Leu Glu Pro Gly Thr Arg Gln Phe Ala Asn Val Pro Val Arg Ser Ser
   1410 1415 1420
   Pro Leu Tyr Glu Tyr Ile Gln Glu Gln Arg Ser Gln Tyr Arg Pro
   1425 1430 1435
<210> 2
   <211> 4320
   <212> DNA
   <213> Mus musculus (house mouse)
<400> 2
   atgtctccac ttataagaag cattgtcgat atcactgaag ttttcaatca atatgcatca 60
   caaagttgtg atggagcatc acttagcaag aaagacctga aaaacctcct tgagagagaa 120
   cttggagatg tccttcagag accacatgac cctgagacga tagacctgac cctagaactt 180
   ctggatcgcg actgcaacgg gcgtgttgat ttcaacgaat tcctcctgtt ccttttcaag 240
   attgctcaag cttgctatta tgctctcgat caggccgcag agctaggcga gaagagagcc 300
   ctgcccaatg aaaagaggaa cctgtcacaa gatcgcaggc aagaagacca aaggagattc 360
   gagccccgaa gcagacaact ggacgaagaa cctgggcgcc gaagctggca gaagagacgt 420
   gagcaggagg agcgcgctga ggagcagcgg ctggagcagc gctacaggca gcaccgcgat 480
   gaagagcaga gactgcaaag gcgagaactg caagaactgg aggaacgcct tgcagagaaa 540
   gagccgcttg gctggagtaa gggtcgtgac gcggaggagt tttctgaggt agaggaacag 600
   caaaggcaag agaggcagga actcaagggc aagggccaaa cagaagagag aaggctgcag 660
   aagcgcaggc aagaagagct acgcgaaccc ctgctaaggc gcgatctgga gttgaggcgc 720
   gaacaagagc taaggcgcga gcaggagttg aggcaggaac agaggcgcga gcaggagcta 780
   aggcgcgagc aggagctgag gcaagagctg aggcgcgagc aggagctgaa tcgaaggcag 840
   gagctgaggc gcgaacaaga gctaaggcgc gagcaggagc tgaggcagga gctgaggcgc 900
   gagcaggagc taaggcgcga gcaggagttg aggcaggaac tgaggcgcga gcaagagcta 960
   aggcgcgagc aggagttgag gcaggaactg aggcgcgagc aagagctaag gcgcgagcag 1020
   gagttgaggc aggaactgag gcgtgagcaa gagctaagac gcgagcaaga gctgaggcag 1080
   gagctggctg aggaggacga gctgacgcgg atccgggaac ccgacgagag cattacccag 1140
   aggtggcagt ggcagctcga aaacgaggca gacgcccgtc agaacaaggt ctactctagg 1200
   cctagcaggc aggagcagag gcttcgccag gagctggggg agcgtcagct ccgggagcag 1260
   gaggagcagc gccgcgacct ccaacaggag cgtcccgctg aggaggcgcg ccagcgcaac 1320
   cagtgggaga ggccgcagcg ggcggaggag cgcctggagc aggagcagcg gttccgcgac 1380
   agggaggagc agcgcttccg ggaggagaag ctgcaacgag cagagctcca ggacagcctc 1440
   ctagatgaag aacagaggcg actccaggag gaacgccgag agccaaacag gagccggcaa 1500
   ctgagggaag aaagccagag gcgccgcaca ctgtacgcca aacccagcca gaggcagcaa 1560
   agaaggcgcc tgcagcagga aaggcagtat caggaggagg acctgcagcg gctgcgggat 1620
   gaagatcagc gcagggatct gaaatggcag tggcaaccaa ggaaagaaaa tgaagttcgt 1680
   agtaacaggc tcttcaccaa acgcagaggg gatgaggaac ccatccagca gctggaagat 1740
   tctcaggagc gagagagacg tcaggatcgg cggcctctgc aagacgaaga ggaagagaag 1800
   agagagctgg agcaggagag gaggcgtcga cagcagcgcg accgtcagat cctagaggaa 1860
   gagcagtttc agcgagagca ccaacgggaa gccagaagac gagatgagac gttccaggag 1920
   gaagaacagc tccagggaga atcgagaaga cggcaacagg agagagaggg caagttcctt 1980
   gaggaggaaa ggcagctgcg gacagaacgg gaagagcaga ggcggcgtca agaacaagag 2040
   agagaattcc aagaggagga ggagcacctc caagaacgcg agaaagaact tcggcaggaa 2100
   tgcgacagaa aatctcgtga acaagagcgc cgccagcagc gtgaggaaga gcagctgagg 2160
   cgtcaggagc gggaccagag attccgtcgg gaacaagaac gccacctgga acgtgaggaa 2220
   gagcagctgc gggacagacc atcccgccgg gaacaagaac gccaccagga acgtgaggaa 2280
   gagcagctgc gggacagacc atcccgccgg gaacaagaac gccaccagga acgtgaggaa 2340
   gagcagctgc gggacagacc atcccgccgg gaacaagaac gccaccagga acgtgaggaa 2400
   gagcagctgc gggacagacc attccgccgg gaacaagaac gccgcctgga gcgtgaggaa 2460
   gagcagctgc gggacagacc atcccgccgg gaacaagaac gccaccagga acgtgaggaa 2520
   gagcagctgc gggacagacc atcccgccgg gaacaagaac gccgcctgga gcgtgaggaa 2580
   gagcagctgc gggacagatc attccgccgg gagcaagagc tcagacggga cagaaaattc 2640
   catgaggaag aagagcgccg cgaggaactg gaggaagagc agcgtggcca agagcgggac 2700
   cgtttgaggg tggaggagca gcttcgcgga cagcgagagg aagagcagcg ccgccgccag 2760
   gaatgtgaca gaaaattaca ccgggaacta gaggtccgcc aggaactgga ggaagagcgg 2820
   ctgcgggaca gaaagctccg cagggaacaa gagctcaggc gcgacagaaa attccatgag 2880
   gaagaagagc gccgtcatga ggagttcgag gaaaagcagc tgcgcctcca ggaaccggac 2940
   agaagattcc gccgggaaca agagctccgt caggaatgcg tcgaggaaga gcggctgcgg 3000
   gacagtaaga tccgccggga gcaagagctc cgccgggagc gcgaagaaga gcggctgagg 3060
   gacagaaaga tccgccggga ccaagaactc cgccagggac tggaggaaga gcagctgagg 3120
   cgccaggaac ttgacagaaa attccgtgag gaacaagagc tcgaccaaga actggaggaa 3180
   gagcggctgc gggacagaaa gatccgccgg gagcaagagc tccgccggga gcaagagctc 3240
   cgccgggagc aagagttccg ccgggagcaa gagctccgcc gggagcaaga gttccgccgg 3300
   gagcaagagc tccgccagga gcgcgaggaa gagcggctga gggacagaaa gatccgccgg 3360
   gaccaagaac tccgccaggg actggaggaa gagcagctga ggcgccagga acgtgacaga 3420
   aaattccgtg aggaacaaga gctcggccaa gaactggagg aagagcggct gcgggacaga 3480
   aagatccgcc gggagcaaga gctccgccgg gaacgcgagc aagagcagcg gcgccgcctg 3540
   gagcgtgagg aagagcagca gcgtctccat gagcgtgagg aagagcagcg gcgccgccag 3600
   gagcgcgagc aagagcagca gcggtgcctg gagcgtgagg aggaacaatt tcgctttgag 3660
   gagcagcagc gccgccgcca ggaacgcgag caacagttga gacaggagcg cgacagaaga 3720
   gtccttgagg aagaagagct tcgtcaggaa agggaggagc tgctgcaccg ccaggtgggt 3780
   ggcaggaaat tccgggaaga ggagcgactc cgcctggaaa gagaggaaca gcagcgtcgt 3840
   ctccaggagc gtgacaacag aagattccgc gaggaagtag agctcaggca agaaagggaa 3900
   gggcagcagc ttcgccaaga gcgtgacaga aaattccgtg aggtagaaga gcttcgccag 3960
   gaagaacagc gccgccgcca ggagcgtgac aggaaattcc gggaagagaa acacccacgc 4020
   gaggaacgcg aggaacagca gttgcgcagg gagaagcgag atggtcaata cctggctgag 4080
   gagcagtttg ccagggatac gattcgtcgc caggaacaag aactacgtca agaagaggaa 4140
   caaagacgtc gccaagagcg ggagagaaaa ttccaagaag agcaaatccg tcgtaggcaa 4200
   gaggagcaga ggcgccgcca aatcctggag cctggtacac gccagtttgc caatgtccca 4260
   gtgcgttcca gccctctcta tgagtacatc caggagcaga ggtctcaata ccgcccttaa 4320
<210> 3
   <211> 30
   <212> DNA
   <213> Synthetic DNA
<400> 3
   atgtctccac ttataagaag cattgtagat 30
<210> 4
   <211> 30
   <212> DNA
   <213> Synthetic DNA
<400> 4
   ttaagggcgg tattgagacc tctgctcctg 30
<210> 5
   <211> 10
   <212> PRT
   <213> Synthetic peptide
<400> 5
   Ser Ile Glu Gln Ser Cys Asp Gln Asp Glu
    1               5                  10